# EUROPEAN PATENT SPECIFICATION

(11) **EP 4 388 553 B1**
(45) Date of publication and mention of the grant of the patent: **07.10.2026**
(21) Application number: 22764726.0
(22) Date of filing: 11.08.2022
(51) Int. Cl.: G16H 50/50, A61B 34/10, A61B 5/367

(54) **IDENTIFICATION OF TARGET SITES FOR VENTRICULAR TACHYCARDIA TREATMENT**
IDENTIFIZIERUNG VON ZIELBEREICHEN FÜR DIE BEHANDLUNG EINER VENTRIKULÄREN TACHYKARDIE
IDENTIFICATION DE SITES CIBLES POUR LE TRAITEMENT DE LA TACHYCARDIE VENTRICULAIRE

(30) Priority: 19.08.2021 EP 21192067
(43) Date of publication of application: 26.06.2024
(73) Proprietor: Medical University of Graz, 8010 Graz (AT); King's College London, London WC2R 2LS (GB); Numericor GmbH, 8010 Graz (AT)
(72) Inventor: PLANK, Gernot, 8010 Graz (AT); BISHOP, Martin, Westminster Bridge Road London SE1 7EH (GB); NEIC, Aurel, 8010 Graz (AT); CAMPOS, Fernando, Westminster Bridge Road London SE1 7EH (GB)
(74) Representative: Maiwald GmbH
(86) International application number: PCT/EP2022/072542
(87) International publication number: WO 2023/020930

(56) References cited:
- WO-A1-2015/153832
- WO-A1-2016/014949
- WO-A1-2016/077154
- WO-A2-2012/109618
- US-A1- 2014 122 048
- LOZOYA ROCIO CABRERA ET AL: "Model-Based Feature Augmentation for Cardiac Ablation Target Learning From Images", IEEE TRANSACTIONS ON BIOMEDICAL ENGINEERING, IEEE, USA, vol. 66, no. 1, 1 January 2019 (2019-01-01), pages 30 - 40, XP011700871, ISSN: 0018-9294, [retrieved on 20181218], DOI: 10.1109/TBME.2018.2818300

## Description

### Technical Field

The present disclsoure relates to a computer-implemented method of determining one or more target sites usable for treatment of ventricular tachycardia. The method may particularly relate to a pre-operative, pre-procedural or intra-procedural method of identifying the one or more target sites. Further, the present invention relates to a computer program for instructing a computing device or system to carry out steps of the method, to a computer-readable medium storing such computer program, and to a correspondingly configured computing device or system.

### Technical Background

Ventricular tachycardia (VT) can result from fibrotic tissue or fibrosis, for example scar tissue or scar-related tissue, within a subject's or patient's heart. Such fibrotic tissue can, for example, be caused by myocardial infarction, coronary heart disease, aortic stenosis, cardiomyopathy, electrolyte problems, a heart attack or another injury or damage of myocardial tissue.

Generally, VT can be associated with locally altered or modified electrical activity of fibrotic tissue with respect to healthy myocardial tissue of the subject's heart. For instance, in an infarcted region of one or more ventricles of a heart, adjacent tissues with altered electrical activity and electrically quiescent infarct scar may form a system of pathways for conduction of electrical impulses. Ventricular tachycardia may result in ventricular fibrillation and may bear a substantial health risk as it can potentially turn into cardiac arrest.

The only curative treatment for patients with incessant VT is radiofrequency ablation (RF), which can involve delivery of energy to targets or target sites, also referred to as ablation sites or ablation target sites, within the fibrotic tissue. For example, a target site at or within fibrotic tissue, for example within a ventricle, can be ablated in order to disrupt the re-entrant electrical excitation that sustains the VT.

This therapy or ablation therapy, however, can be associated with long procedure times and high complication rates, whilst success rates may be comparatively low. Typically, more than 50% of patients will present with VT recurrence within one-year post-procedure.

Failure of ablation therapy can frequently be attributed to an inability to comprehensively identify the myocardial substrate or region within the patient's heart that is capable of sustaining the VT. Attempts to reconstruct the complex intramural propagation pathways through the regions of structural re-modelling to identify targets can be limited due to the surface-nature of catheter-based electroanatomical mapping (EAM) systems combined with inadequate spatial resolution. Furthermore, target site identification during this procedure is usually most accurate when VT is induced, and is for example mapped, during the procedure, which can significantly increase the risk-profile and may often not be haemodynamically tolerated.

The current state-of-the-art in VT ablation relies on EAM systems that combine images and intraprocedural recording of electrograms (EGMs) at many sites of a patient's heart to create a two-dimensional manifold map to characterize the adjacent tissue (Josephson ME, Anter E. "Substrate Mapping for Ventricular Tachycardia Assumptions and Misconceptions", JACC Clin Electrophysiol. 2015;1(5):341-352). Increasingly, EAM systems have the capability to incorporate anatomical information directly from corresponding cardiac magnetic resonance (CMR) or computed tomography (CT) imaging performed prior to the procedure. Such data, usually in the form of segmented shells, may be imported to the system to provide additional information regarding scar and/or fibrosis in the heart, wall thinning in the heart and potentially even identification of channels within scars or fibrotic tissue. Another approach relies on the use of computational models for analyzing the arrhythmogenic substrate seen in images. While these are currently not clinical standard, the initial feasibility of using in-silico image-based patient-specific models has demonstrated to perform virtual VT induction protocols, identify reentrant circuits and subsequently target key ablation sites (Prakosa A, Arevalo HJ, Deng D, et al., "Personalized virtual-heart technology for guiding the ablation of infarct-related ventricular tachycardia", Nat Biomed Eng. 2018;2(10):732-740). The entirely non-invasive nature of these approaches, requiring only magnetic resonanceimaging (MRI), cardiac magnetic resonance (CMR) or computed tomography (CT) imaging data to construct anatomical models, can facilitate pre-procedure planning. Furthermore, the ability to apply 'aggressive' virtual VT-induction protocols from numerous sites within the ventricles, can allow multiple VTs to be probed, increasing the likelihood of inducing the clinical VT (Arevalo HJ, Vadakkumpadan F, Guallar E, et al., "Arrhythmia risk stratification of patients after myocardial infarction using personalized heart models", Nat Commun. 2016;7(May):11437). Once induced, detailed analysis of the ensuing circuits and subsequent re-induction testing may be performed, providing the opportunity to directly test target efficacy. Studies using these approaches have demonstrated close association between the geometrical location of in silico ablation target predictions and clinical lesions associated with acute success in both pre-clinical and clinical retrospective studies.

The clinical translation of electrophysiology-based computational models into a clinical workflow, however, is currently limited, as executing the required simulations is vastly expensive, both in terms of time-frames and the requirement for additional computational resources. Current approaches largely require vast computational resources to perform costly monodomain simulations of cardiac electrophysiology due to the relatively high-resolution of about <350 µm meshes that may be required to ensure adequate convergence of numerical solutions, coupled with the long-duration virtual induction protocols that are performed to identify all potential circuits that may be associated with or result in VT. Typical requirements are estimated at many hours to days using high performance computing (HPC) clusters with access to hundreds of processors or CPUs. Usually, such computing resources are unavailable in clinical environments.

WO 2012/109618 A2 describes methods and devices for patient-specific cardiac treatments, in particular treatment of VT. Magnetic resonance data of a heart are used to simulate electrophysiological or electromechanical activity of the heart. Effects of cardiac treatment, and thus target sites for VT ablation, are simulated by the occurrence of a predefined change in the electrophysiological or electrochemical behavior of the heart. For the actual simulation, high-resolution MR data and a correspondingly powerful pipeline are used to generate the heart model. During the simulation, VT circuits are analyzed to determine optimal target sites for VT ablation. Waves are induced in the heart model at various, unspecified positions and wave break-up and the formation of reentrant waves are simulated.

WO 2016/014949 A1 describes methods and devices for determining target sites for cardiac treatment. This also simulates the propagation of waves in a heart model.

WO 2015/153832 A1 describes methods and devices for patient-specific determination of electrical heart properties. A heart model is represented as a voxel mesh, which is enriched with physiological information relating to the heart tissue.

### Summary

It may, therefore, be desirable to provide for an improved computer-implemented method and a corresponding system, for example addressing, removing or at least mitigating the above-mentioned limitations and drawbacks of current approaches. In particular, it may be desirable to provide for an improved computer-implemented method of determining one or more target sites usable for VT treatment, which can allow for a fast, accurate and reliable determination or identification of potential target sites for VT ablation, while preferably requiring low computational resources.

This is achieved by the subject matter of the independent claims, wherein further embodiments are incorporated in the dependent claims and the following description.

In the following, various aspects as well as embodiments and examples of those aspects of the present disclosure are described. It should be noted that aspects of the present disclosure relate to a computer-implemented method, to a corresponding computer-program, to a computer-readable medium storing such program and to a correspondingly configured computing system or device. It is emphasized, however, that any feature, step, function, element, technical effect and/or advantage described hereinabove and hereinbelow with reference to one aspect of the present disclosure, equally applies to any other aspect of the present disclosure, as described hereinabove and hereinbelow.

According to an aspect of the present disclosure, there is provided a computer-implemented method of determining one or more target sites usable for treatment of ventricular tachycardia, also referred to as VT. The method comprises the following steps:
- receiving, with a computing device or system including one or more processors, three-dimensional model data indicative of a cardiac model modelling an anatomy of a heart of a subject with fibrosis, wherein the cardiac model includes at least one myocardial segment associated with and/or modelled as electrically conducting myocardial tissue and at least one fibrotic segment associated with and/or modelled as insulating fibrotic tissue;
- simulating (referred to as step "S1" herein), by the computing device using the cardiac model, evolution and/or propagation of a cardiac activation wave across the at least one myocardial segment and the at least one fibrotic segment;
- determining and/or computing (referred to as step "S2" herein) at least one split location, at which an isosurface of the simulated cardiac activation wave is split into two or more sections, the at least one split location being indicative of a location for the simulated wave hitting a boundary between the at least one fibrotic segment and the at least one myocardial segment; and
- determining and/or computing one or more target sites based on the determined at least one split location.

As used herein, the cardiac model may refer to a data-driven or data-based model representative or indicative of the anatomy of the heart of a subject, for example an individual or patient. The cardiac model may contain information about one or more of a geometry, a structure, a physiology and/or an electro-physiology of the subject's heart and/or the tissue contained therein.

The cardiac model may comprise a part or portion of the heart related to or constituting the at least one myocardial segment. The at least one myocardial segment may be modelled in the cardiac model as tissue being electrically excitable, for example excitable based on the simulated cardiac activation wave (also referred to as activation wave, or wave herein). In this context, the at least one myocardial segment may be referred to as electrically conducting herein. It is to be noted that any reference herein to one myocardial segment modelled in the cardiac model can include a plurality of myocardial segments.

The cardiac model may further comprise a part or portion of the heart related to or constituting the at least one fibrotic segment. The least one fibrotic segment may be modelled in the cardiac model as fibrotic tissue being electrically unexcitable and/or electrically non-conductive. In this context, the at least one fibrotic segment may be referred to as insulating or electrically insulating herein. It is to be noted that any reference herein to one fibrotic segment modelled in the cardiac model can include a plurality of fibrotic segments.

Such fibrotic tissue may, for example relate to scar tissue or scar-related tissue in the heart. For instance, fibrotic tissue in the patient's heart may be caused by or associated with myocardial infarction, non-ischaemic dilated cardiomyopathy, coronary heart disease, aortic stenosis, cardiomyopathy, electrolyte problems, a heart attack or another injury or damage of myocardial tissue.

According to the present disclosure evolution of a cardiac activation wave is simulated across at least a part of the cardiac model that includes the at least one myocardial segment and the at least one fibrotic segment. For example, evolution and/or propagation of a wave front of the cardiac wave, which may refer to an isosurface of the simulated wave, may be simulated across the cardiac model or at least a part thereof. Therein, evolution of the wave or wavefront may be simulated in time, space or both time and space across the three-dimensional cardiac model. When the cardiac activation wave hits a border, boundary or boundary region between the myocardial segment and the fibrotic segment, the wavefront or another isourface of the wave may be split into two sections or portions, due to the changing electrical properties of the myocardial segment and the fibrotic segment. The split location may generally be given as or indicative of a point, location or region in three-dimensional space, at which the wavefront and/or an isosurface of the cardiac activation wave is split into two or more sections. The split location may be indicative of or contain coordinates in three-dimensional space, for example in a reference coordinate system of the cardiac model or any other coordinate system. It is to be noted that any reference herein to one split location can comprise a plurality of split locations.

Based on the at least one split location, the location of the fibrotic segment and/or a boundary between the fibrotic and the myocardial segment may be reliably and accurately determined. As the fibrotic segment may result in a VT, one or more target sites for treatment of VT can be determined or identified based thereon. It is to be noted that any reference herein to one target site can comprise a plurality of target sites. The at least one target site can be derived from or computed based on the determined one or more split locations. Optionally, this can include selecting one or more split locations as target site. A target site may generally be given as or indicative of a point, location or region in three-dimensional space, which may be involved in, associated with or causing VT. The target site may be indicative of or contain coordinates in three-dimensional space, for example in a reference coordinate system of the cardiac model or any other coordinate system.

In an example, the determined one or more target sites can be used in a subsequent procedure or treatment of VT, for example a VT ablation treatment, e.g. using a catheter-based electroanatomical mapping (EAM) system or other treatment device. Therein, the one or more target sites or corresponding spatial coordinates may refer to or denote a site, location point or region of the heart that may be ablated in a subsequent treatment, i.e. a treatment subsequent to performing the method of the present disclosure, in order to efficiently treat the VT. For example, spatial coordinates indicated by the target site and/or spatial coordinates derived therefrom can be supplied to an EAM system for VT treatment.

In this context, the method according to present disclosure can be considered as pre-operative or pre-procedural computer-implemented method, for example a treatment planning method. Accordingly, the invention or present disclosure does not involve or in particular comprise or encompass an invasive step which would represent a substantial physical interference with the body of the subject or patient requiring professional medical expertise to be carried out and entailing a substantial health risk even when carried out with the required professional care and expertise. Particularly, the invention does not involve, comprise and/or encompass any surgical or therapeutic activity. The invention is instead directed as applicable to treatment planning before carrying out the actual treatment on the patient or subject. For this reason alone, no surgical or therapeutic activity and in particular no surgical or therapeutic step is necessitated or implied by carrying out the method according to the present disclosure. It is noted, however, that the method according to the present disclosure can also be used as intra-procedural method.

As described above, according to the present disclosure, one or more target sites for VT treatment can be determined based on simulating a cardiac activation wave across or through a cardiac model and based on determining the one or more split locations of the wave. Therein, simulating a cardiac activation wave may include or refer to simulating a propagation wave front. Generally, such simulation can be performed in time-efficient and cost-efficient manner, for example requiring minimum personnel and computing resources. In particular, the method according to the present disclosure can be performed in real-time or near real-time on a regular computer or computing device, such as a desktop computer. It is to be noted, though, that the present disclosure is not limited to use of a regular computer or computing device, but comprises use of any computing device, including smart phones, tablet PCs, cluster PCs, computing systems with a plurality of computing devices, computing networks, cloud servers, and the like. Accordingly, the method of the present disclosure can provide for a procedure that is lightweight in computational cost, that can be executed in real-time or near real-time, for example using a standard computer desktop machine, and that can even be used as an online tool. Also, the method may be semi-automated or even fully automated.

The method of the present disclosure can be in particular beneficial when compared to conventional approaches, in which electrophysiological or electromechanical activity of the subject's heart is simulated, which can be associated with large computational effort. In contrast thereto, the method of the present disclosure can be considered as being based on a topological detection of the target site, which can be executed in real-time or near real time.

According to an embodiment, the at least one fibrotic segment is traversed by one or more conductive channels, for example one or more conductive channels formed by, in and/or within fibrotic tissue. The one or more conductive channels may be formed within and/or may be part of ventricles (e.g. one or both ventricles) of a heart. For instance, in an infarcted region of one or more ventricles of a heart, adjacent tissues with altered electrical activity and electrically quiescent infarct scar may form a system of one or more pathways and/or channels for conduction of electrical impulses. Further, the at least one split location can be indicative of a location for the simulated wave entering into or exiting from one of the one or more conductive channels in the fibrotic segment. Accordingly, based on determining the split location an entry of the cardiac activation wave into the one or more conductive channels and/or an exit of the wave out of one or more conductive channels can be reliably determined or detected.

As described above, VT may result from or be associated with a re-entrant electrical excitation or activation of the subject's heart, which may result in a comparatively high heart rate superimposing the subject's regular heartbeat. When simulating evolution of the cardiac activation wave, such re-entrant electrical excitation may result in an electrical circuit or be simulated as electrical circuit, for example a closed electrical circuit or loop-like circuit partially or entirely encompassing the fibrotic segment and/or partially traversing the fibrotic segment, for instance through the one or more conductive channels. Hence, by determining the one or more split locations, regions or portions of the heart may be identified, which may potentially allow for a re-entrant electrical excitation that may potentially result in VT, and hence may be a potential candidate for a target site usable for treatment of VT.

According to an embodiment, determining the one or more target sites based on the determined at least one split location further comprises simulating evolution of at least one further cardiac activation wave unidirectionally excited at the at least one split location. As used herein, a unidirectionally excited cardiac activation wave may refer to an activation wave propagating from the site of stimulus, for example the split location, in one spatial direction, whereas propagation of the wave in one or more further directions may be blocked. Such unidirectionally excited wave may for example travel along a circuit, electrical circuit or loop, which may indicate that the corresponding split location, at which the wave was excited, may be a target site usable for VT treatment. Accordingly, the method may comprise determining whether the unidirectionally excited at least one further wave travels along a circuit, electrical circuit, a closed loop circuit and/or a loop through the cardiac model. This may mean that the excited activation wave arrives at the site of excitation after a certain travel time, which is also referred to as round trip time herein.

According to an embodiment, a plurality of split locations is determined, wherein for each of the split locations at least one further cardiac activation wave unidirectionally excited at the respective split location is simulated. By simulating at least one further activation wave at each determined split location, it may be reliably and efficiently determined whether the respective split location might be part of a circuit or electrical circuit that could sustain a VT, thereby allowing to reliably determine one or more target sites usable for VT treatment.

According to an embodiment, a plurality of split locations is determined, wherein the method further comprises discarding at least one of the determined split locations based on determining that said at least one split location is arranged between two neighboring split locations on opposing sides of said at least one split location. Optionally, this may include determining that said split location and the two further split locations arranged on opposite sides thereof are part of an electrical circuit, such as a closed circuit or loop-like circuit, interconnecting the three split locations.

For example, one of the split locations may be arranged at an entry of a conductive channel traversing the fibrotic segment, one split location may be arranged at an exit of the conductive channel, and one split location may be arranged within the conductive channel between the other two split locations. While all three split locations may be usable as target sites for VT treatment in general, it may be favorable to select the split location at the entry and/or the one at the exit of the conductive channel as target site(s), as ablation of tissue at one of these locations in a subsequent procedure may efficiently cure VT and be better accessible during the treatment, when compared to the split location that is arranged within the conductive channel.

According to an embodiment, the method further comprises determining, for each of the determined split locations, an activation time map (also referred to as activation map) indicative of a spatio-temporal evolution of the simulated at least one further wave across the cardiac model. Alternatively or additionally, simulating the at least one cardiac activation wave and/or the at least one further cardiac activation wave may comprise simulating spatio-temporal evolution of the corresponding cardiac activation wave through the cardiac model. As used herein, an activation map or activation time map may be indicative of or refer to the activation or excitation of myocardial tissue as a function of space and time in response to excitation of the respective cardiac wave at one or more stimulus sites.

According to an embodiment, the method further comprises determining a round trip time based on the simulated at least one further cardiac activation wave, the round trip time being indicative of a travel time of the at least one further cardiac activation wave along an electric circuit between initiation or excitation on one side of the at least one split location and arrival at an opposite side of the at least one split location. Alternatively or additionally, the method further comprises determining a round trip distance or pathlength based on the simulated at least one further cardiac activation wave, the round trip distance or pathlength being indicative of a length of an electric circuit, along which the at least one further activation wave travels from one side of the at least one split location to an opposite side of the at least one split location. Optionally, it may be determined whether the unidirectionally excited at least one further cardiac activation wave travels along a circuit or electrical circuit, for example a loop-like circuit encompassing or comprising the at least one split location, at which the at least one further cardiac activation wave was excited.

Accordingly, by determining the round trip time and/or the round trip path, it may be reliably determined whether the corresponding split location is part of an electrical circuit that could potentially allow for re-entrant electrical excitation and hence sustain VT. For instance, the round trip time and/or the round trip distance may be indicative of a size of the corresponding electrical circuit, which can allow to estimate or determine whether the electrical circuit is large enough to result in or sustain VT. As a consequence, the one or more target sites usable for VT treatment can be determined accurately.

According to an embodiment, the method further comprises comparing the determined round trip time and/or the determined round trip distance to a threshold value. The method further comprises selecting the at least one split location as target site if the determined round trip time and/or the determined round trip distance reaches or exceeds the threshold value. Alternatively or additionally, the split location may be discarded based on determining that the determined round trip time and/or the determined round trip distance is below the threshold value. As noted above, for sustaining VT, a sufficiently large electrical circuit allowing for re-entrant electrical excitation of myocardial tissue is usually required. Hence, by comparing the round trip time and/or the round trip distance to one or more corresponding threshold values, this criterion may efficiently be taken into account for determining the one or more target sites usable for VT treatment.

It should be noted that in the context of the present disclosure one or both, the round trip time and the round trip distance can be used, in particular since either of these quantities may be converted into the other, for example when a velocity of the simulated activation wave through the fibrotic segment and the myocardial segment is known or can be approximated.

According to an embodiment, a plurality of split locations is determined and for each of the determined split locations a round trip time and/or a round trip distance is determined, wherein the at least one target site is determined based on selecting the split location according to one or more criteria related to the round trip time and/or the round trip distance. A criterion may, for example, be a maximum of the round trip time and/or the round trip distance. In other words, the split location associated with the largest round trip time and/or distance among the determined round trip times or distances may be selected as target site. Alternatively or additionally, the split location associated with the smallest round trip time and/or distance may be selected as the target site. Also other criteria, such as a median or average value of the round trip time and/or distance may be used to select the target site.

According to an embodiment, the method further comprises simulating, by the computing device, a unidirectional conduction block in the cardiac model at the at least one split location to block traversal of the at least one further cardiac activation wave at the at least one split location in at least one spatial direction, in particular in a direction opposite to a direction, in which the further wave was excited. In other words, unidirectional propagation of the at least one further cardiac activation wave may be simulated by unidirectionally exciting the wave at one split location and by blocking propagation of the wave in one or more other directions, in particular in opposite direction. This may allow to efficiently determine whether the excited wave travels along an electrical circuit from one side of the split location to an opposite side thereof. Consequential thereto, the target site usable for VT treatment can be reliably detected or determined.

According to an embodiment, the cardiac model is based on a spatio-temporally discretized model. For example, the cardiac model may include or refer to a finite element model, a finite volume model, or a finite difference model. Also other models, such as a meshless model, may be used as cardiac model. Further, the unidirectional conduction block may be simulated based on simulating an isolated surface at the at least one split location. For example, the unidirectional conduction block may be simulated based on decoupling one or more elements of a finite element model, a finite volume model or a finite difference model at the at least one split location.

According to an embodiment, the cardiac activation wave and/or at least one further cardiac activation wave is simulated based on stimulating spatio-temporal evolution and/or propagation of the respective activation wave at one or more stimulus sites in the cardiac model. Alternatively or additionally, the cardiac activation wave and/or at least one further cardiac activation wave is simulated based on a wavefront propagation model and/or based on a model representing arrival times and/or travelled distances of propagating waves or wavefronts. For example, an Eikonal model, a Reaction Eikonal model, a Reaction Diffusion model, a combination thereof and/or any other model able to represent or representing the spatio-temporal evolution of propagation waves, such as a marching cubes model, may be used for simulating the one or more cardiac activation waves.

According to an embodiment, a plurality of split locations is determined and the method further comprises determining, for each of the determined split locations, an activation time map indicative of a spatio-temporal evolution of the simulated at least one further wave across the cardiac model. Further, the method may comprise computing a correlation coefficient between temporally aligned activation time maps associated with different split locations, and determining the one or more target sites based on comparing the computed correlation coefficients to a threshold value. Therein, temporally aligned may mean that one or both the round trip times and the round trip distances calculated for different split locations are normalized, such that the corresponding cardiac activation waves had the same start location and/or the same start time.

By determining the correlation coefficients for different activation time maps associated with different split locations, it may be determined which of the split locations are part of the same or a similar electrical circuit. Accordingly, it may be determined which of the electrical circuits and the corresponding split locations are unique from one another, represent distinct anatomical pathways and/or most likely to sustain VT. Using such "unique" split location (or split location associated with a unique circuit) as target site for the subsequent VT treatment may be most effective in the treatment or may most likely allow to cure the VT.

According to an embodiment, the method further comprises generating an updated cardiac model based on permanently blocking traversal of a cardiac activation wave at the target site. Further, the method comprises repeating at least the step of simulating, by the computing device using the updated cardiac model, evolution of a cardiac activation wave across the at least one myocardial segment and the at least one fibrotic segment, and determining at least one split location, at which an isosurface of the simulated cardiac activation wave is split into two or more sections when travelling across the updated cardiac model. By permanently blocking traversal of the cardiac activation wave at the target site, ablation of tissue at the respective target site can be simulated, thereby allowing to determine a degree of effectiveness of a subsequent VT treatment at the target site. In other words, it may be tested whether ablation of tissue at the target site can prevent re-entrant excitation at the target site and hence can terminate or cure VT.

When the cardiac model is based on a finite element, finite volume or finite difference model, traversal of the cardiac activation wave may be blocked and/or the updated cardiac model may be generated based on disconnecting one or more nodes or elements of the model associated with the target site.

According to an embodiment, the method further comprises determining an electric potential field induced by the simulated cardiac activation wave, for example thereby generating electrogram data indicative of an electrocardiac and/or electrical effect of the simulated cardiac activation wave. Such simulation may be of particular advantage for the subsequent VT treatment, as such simulations may be inter-compared with actual measurements on the subject during treatment.

According to an embodiment, the method further comprises receiving three-dimensional imaging data of the heart and segmenting the imaging data into the at least one myocardial segment and the at least one fibrotic segment. Such imaging data may for example relate to magnetic resonance imaging data and/or computed tomography imaging data. However, also data from other imaging modalities may be used. The method further comprises assigning space- and direction-dependent conduction velocities of travelling wave fronts to the cardiac model. For example, fiber orientations of myocardial fibers can be associated to the at least one myocardial segment and/or the myocardial tissue contained therein.

A further aspect of the present disclosure relates to a computer program, which, when executed on a processor of a computing device or system, instructs the computing device or system to carry out steps of the method, as described hereinabove and hereinbelow.

A further aspect of the present disclosure relates to a non-transitory computer-readable medium storing a computer program, which, when executed on a processor of a computing device or system, instructs the computing device or system to carry out steps of the method, as described hereinabove and hereinbelow.

A further aspect of the present disclosure relates to a computing system or device with one or more processors, wherein the computing system or device is configured to carry out steps of the method, as described hereinabove and hereinbelow.

It is emphasized that features, functions, elements and/or steps, which are described above and in the following with reference to one aspect of the invention, equally apply to any other aspect of the invention described above and in the following. Particularly, features and/or steps, as described above and in the following with reference to the method, equally apply the computer program, to the computer-readable medium, and to the computing system or device, and vice versa.

These and other aspects of the present disclosure will be apparent from and elucidated with reference to the exemplary embodiments described hereinafter.

### Brief Description of the Drawings

In the following, the present disclosure is described with reference to the appended figures which give background explanations and represent exemplary embodiments of the invention. The scope of the invention is however not limited to the specific features disclosed in the context of the figures.
Fig. 1 shows a computing system for determining one or more target sites usable for treatment of ventricular tachycardia according to an exemplary embodiment.
Fig. 2 shows a flowchart illustrating steps of a method of determining one or more target sites usable for treatment of ventricular tachycardia according to an exemplary embodiment.
Figs. 3 to 6 each illustrate a cardiac model and illustrate steps of a method of determining one or more target sites usable for treatment of ventricular tachycardia according to an exemplary embodiment.

The figures are schematic only and not true to scale. In principle, identical or like parts, elements and/or steps are provided with identical or like reference symbols in the figures.

### Detailed Description of Exemplary Embodiments

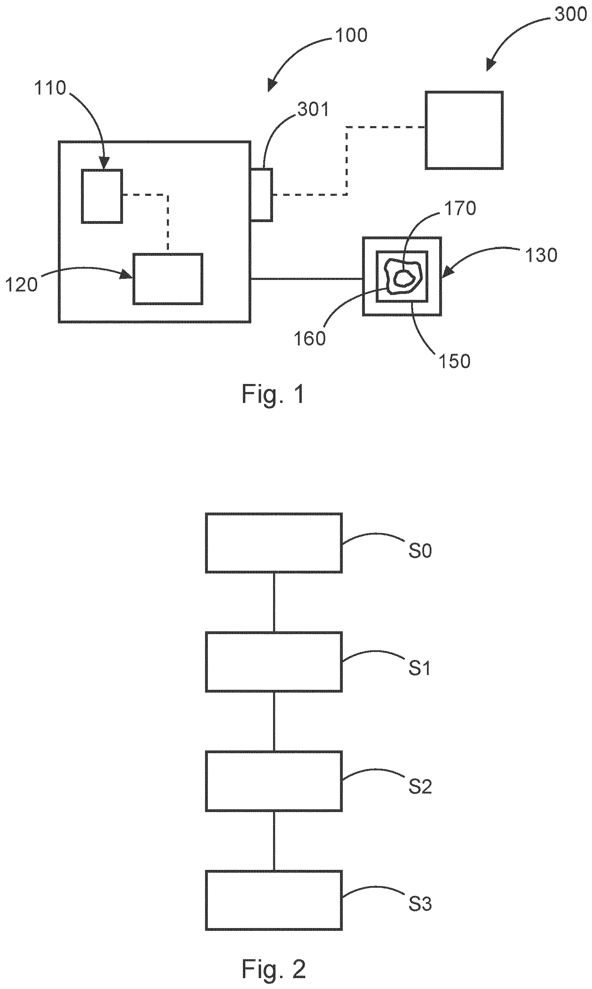

Figure 1 shows a computing system or device 100 for determining one or more target sites usable for treatment of ventricular tachycardia according to an exemplary embodiment.

The system 100 comprises one or more processors 110 for data processing and a data storage 120 for storing data. The data storage 120 may contain software instructions or a computer program, which, when executed by the one or more processors 110 instructs the computing system 100 to carry out steps of the method of determining one or more target sites usable for treatment of VT, as described hereinabove and hereinbelow.

The computing system 100 further comprises a user interface 130 allowing to operatively control the computing system 100, for example by a user or operator, and/or allowing to display data or information, such as a cardiac model 150.

The computing device or system 100 is configured to receive and/or process three-dimensional model data indicative of the cardiac model 150, the cardiac model 150 modelling an anatomy of a heart of a subject with fibrosis. As will be described in more detail with reference to subsequent figures, the cardiac model 150 includes at least one myocardial segment 160 associated with and/or modelled as electrically conducting myocardial tissue and at least one fibrotic segment 170 associated with and/or modelled as insulating fibrotic tissue. The cardiac model 150 or corresponding model data may be stored at the storage device 120 and retrieved therefrom. Alternatively or additionally, the cardiac model 150 may be received from another data source, for example via a communication interface of the computing system 100.

The computing system 100 is further configured to simulate, based on or using the cardiac model 150, evolution of a cardiac activation wave across the at least one myocardial segment 160 and the at least one fibrotic segment 170. Further, the computing system 100 is configured to determine at least one split location (not shown), at which an isosurface, for example a wavefront, of the simulated wave is split into two or more sections, the at least one split location being indicative of a location for the simulated wave hitting a boundary between the at least one fibrotic segment 170 and the at least one myocardial segment 160. Moreover, the computing system 100 is configured to determine one or more target sites (not shown) based on the determined at least one split location.

The computing system 100 may optionally be coupled to or may be couplable to a treatment device 300 for treating VT based on the determined one or more target sites. The treatment device 300 may be coupled to the computing system 100 via an interface 301, which may be a wired interface 301 or a wireless interface 301.

Figure 2 shows a flowchart illustrating steps of a method of determining one or more target sites usable for treatment of ventricular tachycardia according to an exemplary embodiment.

Step S0 comprises receiving, with a computing device or system 100 including one or more processors 110, three-dimensional model data indicative of a cardiac model 150 modelling an anatomy of a heart of a subject with fibrosis, wherein the cardiac model 150 includes at least one myocardial segment 160 associated with and/or modelled as electrically conducting myocardial tissue and at least one fibrotic segment 170 associated with and/or modelled as insulating fibrotic tissue.

Step S1 comprises simulating, by the computing device or system 100 using the cardiac model 150, evolution of a cardiac activation wave across the at least one myocardial segment 160 and the at least one fibrotic segment 170.

Step S2 comprises determining at least one split location (see subsequent figures), at which an isosurface, for example a wavefront, of the simulated wave is split into two or more sections, the at least one split location being indicative of a location for the simulated wave hitting a boundary between the at least one fibrotic segment 170 and the at least one myocardial segment 160.

Step S3 comprises determining one or more target sites (see subsequent figures) based on the determined at least one split location.

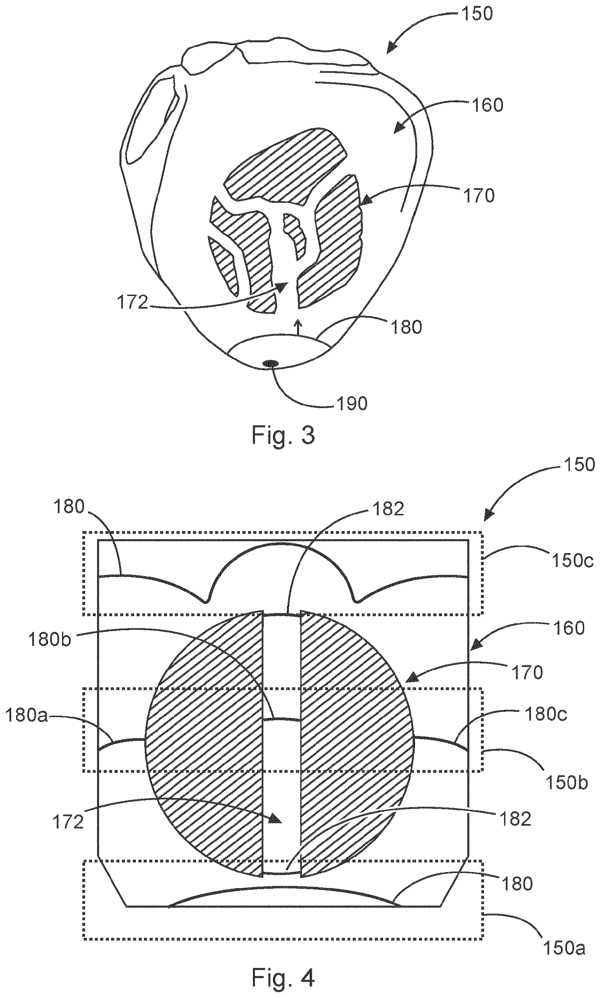

Figures 3 to 6 each illustrate a cardiac model 150 and illustrate steps of a method of determining one or more target sites usable for treatment of ventricular tachycardia according to an exemplary embodiment. In the following, it is referred to one or more of these figures.

The cardiac model 150 may be generated based on segmenting three-dimensional imaging data of the heart into the at least one myocardial segment 160 and the at least one fibrotic segment 170. Such imaging data may for example relate to magnetic resonance imaging data and/or computed tomography imaging data. However, also data from other imaging modalities may be used. Further, space- and direction-dependent conduction velocities of travelling cardiac activation wave fronts may be assigned to the cardiac model 150 in order to model the myocardium segment 160 as electrically conducting myocardial tissue and in order to model the fibrotic segment 170 as insulating fibrotic tissue. For example, fiber orientations of myocardial fibers can be associated to the at least one myocardial segment 160 and/or the myocardial tissue contained therein.

For example, the cardiac model 150 may be generated or constructed as a bi-ventricular model from a whole heart, end diastolic, three-dimensional, steady state free precession (MRI or CT) imaging data with an isotropic resolution between 0.5 mm and 2 mm, for example about 1.3 mm. Tetrahedral finite elements with a mean edge of about 1mm, for example about 976 µm discretization, may be used to construct or generate the cardiac model 150. Myocardial fiber orientations may be incorporated into the cardiac model 150 or associated to the myocardial segment 160 using a rule-based approach. Further, the fibrotic segment 170 may be modelled, for example, based on assigning scar or fibrotic tissue transcended by a network of one or more electrically conductive channels 172 to the cardiac model 150.

Alternatively or additionally, the cardiac model 150 may be generated based on late-gadolinium enhanced cardiovascular MRI with an isotropic voxel resolution of about 1 mm. MRI imaging data or scans may be converted into the cardiac model 150. This may include segmenting myocardium and blood pool. A predefined signal intensity range of the MRI imaging data within the segmented myocardium may be used to calculate a threshold for the myocardial segment 160, such as a threshold of about 60% of the maximum intensity within an MRI image, and a corresponding threshold for the fibrotic segment 170, which may be about 40% of the maximum intensity within an MRI image.

The cardiac model 150 may be based on a spatio-temporally discretized model, such as a finite element, a finite volume or finite difference model. For example, a tetrahedral finite element model may be used based on the segmented MRI or CT data. Such cardiac model 150 may have a comparatively high resolution, for example with an average finite element edge length of about 250 to 700 µm, for example about 340 µm. The meshes of the model 150 may optionally be downsampled, for example to about 750 µm to 950 µm. It is emphasized that such cardiac model 150 is illustrative and exemplary only. The present disclosure is not limited to such model.

To determine one or more target sites, evolution and/or propagation of one or more cardiac activation waves may be simulated. In Figures 3 and 4, a wavefront 180 or isosurface 180 of a cardiac activation wave is stimulated at a stimulus site 190 and propagates through the cardiac model 150, as indicated by the arrow in Figure 3. Therein, wavefront 180 may refer to an isosurface of the cardiac activation wave, i.e. a surface of equal cardiac activation.

Generally, a target site may be determined based on finding an isosurface of the cardiac wave associated with a simulated activation wavefront 180 that splits at a boundary of the myocardial segment 160 and the fibrotic segment 170 into two or more sections 180a, 180b, 180c, as illustrated in Figure 4. As shown in this Figure, when the fibrotic segment 170 contains one or more conductive channels 172, for example within a ventricle, the activation wave or the corresponding isosurface 180 may be split into sections 180a, 180b, 180c at one or more split locations 182 due to the presence of isolating fibrotic tissue, for example due to surviving tissue within a scar generating an isthmus. Such isthmuses provide isolated conduction pathways or channels 172 and are connected to the healthy myocardium at entrance and exit sites.

As illustrated in Figure 4, in the absence of an isolated isthmus or when traversing the myocardial segment 160 only in region 150a of the cardiac model 150, the cardiac activation wave comprises a single connected isosurface. In region 150b of the cardiac model 150, the wave encounters both the myocardial segment 160 and the fibrotic segment 170 with conductive channel 172. When the wave or wavefront 180 encounters the conductive channel 172, the isosurface or wavefront 280 splits into two or more components or sections 180a, 180b, 180c, wherein a smaller section 180b may be formed within the the isthmus or channel 172 itself, which is unconnected to the larger sections 180a, 180c of the isosurface wrapping around the rest of the fibrotic segment 170 due to the nonconducting fibrotic tissue at its boundaries. By locating the points or regions in space, at which splitting occurs, i.e. appears and/or disappears, it may be determined where the wave enters into the channel 172 (see region 150a of cardiac model 150 in Figure 4) and/or exits the channel 172 (see region 150c of cardiac model 150 in Figure 4). The locations, points or regions, at which splitting occurs, are referred to as split locations 182 herein. Once a split location 182 is determined, for example the mesh of the cardiac model 150 may be split along its respective isosurface to create a region of unidirectional conduction block. Cardiac activation may then be simulated with one or more further cardiac activation waves, for instance using a fast Eikonal model or other wavefront propagation model, as will be described in more detail in the following.

For example, a distance field may be computed from a source point or stimulus site 190. The distance field may depend on the location of the stimulus, the fiber directions within the cardiac model 150 and on the anatomy of the three-dimensional fibrotic tissue.

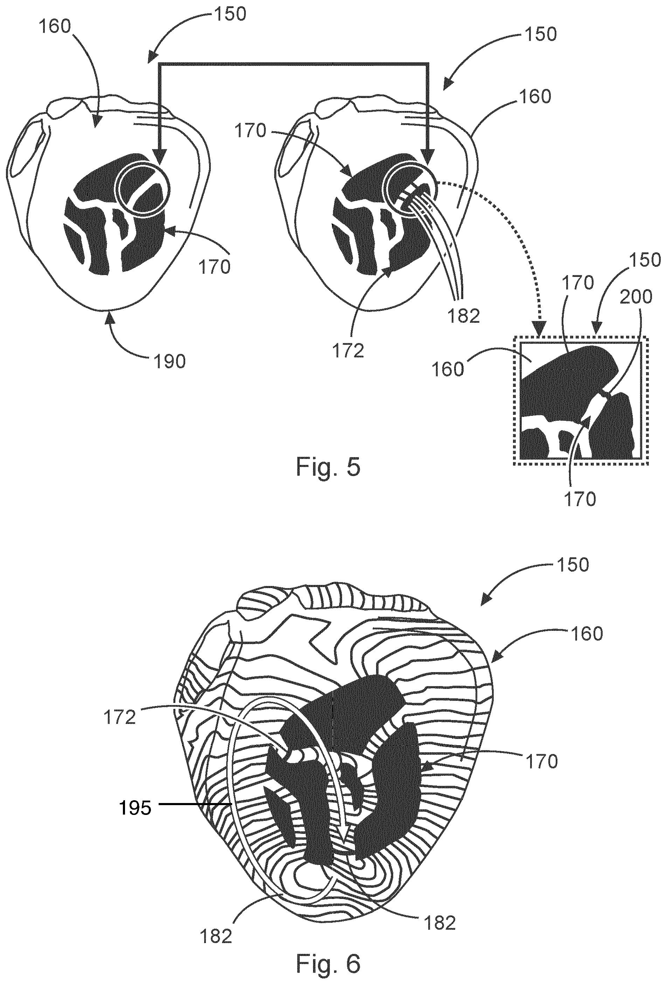

Figure 5 shows on the left-hand side an exemplary distance field computed by stimulating the cardiac activation wave at the apex 190 of the heart. The split locations 182, i.e. the locations 182 where isosurfaces or wavefronts 180 of the cardiac activation wave split are shown in the center or middle illustration of Figure 5 and are encircled in Figure 5 for illustration purposes. All split locations 182 are in fact three-dimensional across the ventricular wall or channel 172.

When using the cardiac model 150 with the fibrotic segment 170 illustrated in Figures 3 to 6, a plurality of split locations 182 may be determined, each corresponding to or being associated with one or more of the conductive channels 172 within the fibrotic segment 170.

To determine one or more target sites based on the detected split locations 182, only those split locations 182 without downstream or without upstream neighbors (with respect to propagation of the activation wave) may be selected. Such split locations 182 may refer to locations where the cardiac activation wave enters into or exits from one of the channels 172. Accordingly, such split locations 182 may represent a location or region where the specific isosurface is generated by splitting into the segments 180a-c or is terminated due to recombination of the segments 180a-c.

Further, a VT may be simulated or induced based on simulating one or more further cardiac activation waves unidirectionally excited at the one or more determined split locations 182. For instance, a VT may be induced or simulated using an Eikonal model, a Reaction Eikonal model, a Reaction Diffusion model or any other model of wavefront propagation.

In an example, the split locations 182 indicative of exit sites for the activation wave exiting from one of the channels 172 may be selected, as described above. Once all split locations or sites for a given distance field are detected, it may be checked whether they can sustain VTs. This can be achieved by simulating one or more further activation waves unidirectionally excited at the respective split location 182, for example using a unidirectional conduction block at the respective split location, which blocks traversal of the activation wave through the respective split location 182 in a direction opposite to the direction, in which the wave was excited or initiated. Such unidirectional block can be created, for example, by splitting the mesh of the cardiac model 150 along an isosurface associated with the respective split location 182, thereby creating a region of disconnected tissue across which activation cannot travel. Mesh splitting can be done using a discontinuous finite element approach that imposes electrical insulation along edges or faces of finite elements. Therein, elements lying on the isosurface can be marked for insulation, and insulation can be enforced by decoupling nodes shared between adjacent finite elements. Shared nodes can then be duplicated, with one set located on one side of the insulating surface, while the other set, with same spatial coordinates, can be assigned at the other side of the isosurface. Cardiac activation or the one or more further cardiac activation waves may then be initiated on a set of nodes on one side of the decoupled surface that corresponds to the respective split location 182, which can ensure unidirectional block or blockage of the wavefront that propagates along a single direction along the isthmus or channel 172.

An activation time map may be determined for the at least one further cardiac activation wave, which time map is indicative of spatio-temporal evolution of the at least one further cardiac activation wave through the cardiac model 150. Figure 6 shows an exemplary activation time map associated with a split location 182 or exit site detected in response to exciting the at least one further cardiac activation wave near the apex of the heart. Activation was initiated at nodes of the cardiac model 150 located at the split location 182 (or exit site) near the apex. Isolines or iso-curves represent the (arrival) time after initiation of the activation.

Further, a round trip time and/or a round trip distance may be determined for the at least one further cardiac activation wave, for example based on the determined activation time map. Therein, the round trip time may be indicative of the time the wavefront takes to travel back to the region of the unidirectional block or respective split location 182. Likewise, the round trip distance may be indicative of the corresponding pathlength of the activation wave or the distance the wave travels.

As discussed above, VT can usually be attributed to re-entrant electrical excitation of the cardiac activation wave, which then travels along an electrical circuit starting on one side of the split location 182 and arriving at an opposite side thereof. Such electrical circuit is exemplary shown in Figure 6 by the circular arrow 195.

Optionally, VT dynamics and electrocardiograms (ECG) may be simulated, for example allowing for a quick investigation of the electrical wavefront and ECG associated with the VT. This may be achieved by solving a model, such as for example the reaction-Eikonal model, representing electrical sources associated with wavefront propagation and the electric potential fields linked to these sources. Alternatively or additionally, a Reaction Diffusion model may be used for this purpose. Therein, cardiac depolarization may be computed in the Reaction Eikonal model by triggering the reaction model through the injection of stimulus currents in the Reaction Diffusion model at time instants given by a prescribed activation time map, or by using a template-based time shifted model of the electrical source. The reaction part of both Reaction Eikonal and Reaction Diffusion models can be simulated by solving a computational model of the cardiac action potential, such as the ten Tusscher model (K.H.W.J. ten Tusscher and A.V. Panfilov, "Alternans and spiral breakup in a human ventricular tissue model.," Am. J. Physiol. Heart Circ. Physiol., 291(3):H1088-100, 2006.). Using the Reaction Eikonal model may provide a fast way to obtain electrograms and ECGs of the VT for clinical comparison. ECGs can be obtained here from electrograms calculated from leads located at approximated clinical standard electrode positions, for example using a model of the torso surrounding the heart. Additional cycles of VT dynamics can be simulated to obtain characteristic ECG complexes as a unique signature of a given VT.

It is noted that Figure 6 exemplary shows or illustrates one simulated VT. Different VTs can be induced depending on the distance field, or pacing location, in a clinical setting, for example. It is noted that the at least one further cardiac activation wave simulated as described above can be excited at a single stimulus site 190 or at a plurality of different stimulus sites 190. The latter may allow to detect all possible split locations 182 that may result in a VT.

To determine one or more target sites 200 usable for VT treatment, as exemplary shown on the right-hand side of Figure 5, the round trip time and/or the round trip distance determined for said split location may be compared to a predefined threshold value for the round trip time and/or the round trip distance. For instance, a threshold value of about 50 ms for the round trip time may be used and only split locations 182, at which cardiac waves with at least 50 ms round trip time can be excited, may be selected as target sites 200, as these may result in re-entrant electrical excitation, for example along circuit 195, as illustrated in Figure 6. However, other threshold values for round trip time and/or distance may be chosen.

Further, uniqueness of a corresponding VT may be determined. For this purpose, it may be determined, which VTs (simulated by unidirectional excitation of one or more further cardiac activation waves), are unique among all induced ones. This step may allow to determine similar exit sites or split locations 182, i.e. split locations 182 resulting in similar VTs, which may be considered as different VTs with subtly different reentrant pathways or circuits 195. For this purpose, correlation coefficients between activation time maps determined for different split locations 182 may be used to assess uniqueness. Specifically, activation time maps may be cyclically aligned prior the correlation calculation, based on determined round trip time and/or round trip distance, such that they had the same starting location and/or starting time. The determined correlation coefficients may be compared to a threshold value, and for example VTs or the corresponding split locations 182 with a correlation coefficient smaller than or equal to the threshold may be selected as target sites 200, whereas VTs or the corresponding split locations 182 with coefficients larger than the threshold value may be considered redundant. For example, a threshold between 0.6 and 0.95, such as about 0.8, may be chosen.

Further, actual ablation of tissue at the determined target sites 200 may be simulated in order to determine potential effectiveness of the subsequent treatment. For example, a list of target sites 200 may be determined, which may be associated with unique VTs. Such list may be sorted based on the round trip times and/or round trip distances determined for each of the target sites 200, for example in a descending order. The first target site 200 in the list associated with the first VT in the list may be marked for ablation. The cardiac model 150 may then be "ablated", for example by disconnecting the nodes associated with finite elements belonging to the target site 200, thereby creating an updated cardiac model 150. Using the updated cardiac model 150, the process may be repeated, i.e. all split locations 182 may be determined and based thereon all target sites 200 may be determined and gathered in an updated list that may optionally be sorted in descending order according to the round trip times and/or distances. This step aims to detect all unique circuits 195, as for example illustrated Figure 6. If further split locations 182 can be found using the updated (or ablated) cardiac model 150, the target site 200 associated with the next VT in the list can be marked for ablation and the method can be iteratively repeated until no split surface or split location 182 is found anymore. This can result in one or more target sites 200 with high potential for curing VT, when used as ablation sites in a subsequent treatment.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims. The invention is as defined in the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A computer-implemented method of determining one or more target sites (200) for planning treatment of ventricular tachycardia, the method comprising:
receiving, with a computing device (100) including one or more processors (110), three-dimensional model data indicative of a cardiac model (150) modelling an anatomy of a heart of a subject with fibrosis, wherein the cardiac model (150) includes at least one myocardial segment (160) associated with and/or modelled as electrically conducting myocardial tissue and at least one fibrotic segment (170) associated with and/or modelled as insulating fibrotic tissue;
simulating (S1), by the computing device (100) using the cardiac model (150), evolution of a cardiac activation wave across the at least one myocardial segment (160) and the at least one fibrotic segment (170);
**characterized in that** the method further comprises:
determining (S2) at least one split location (182), at which an isosurface (180) of the simulated wave is split into two or more sections (18a-c), the at least one split location (182) being indicative of a location for the simulated wave hitting a boundary between the at least one fibrotic segment (170) and the at least one myocardial segment (160); and
determining one or more target sites (200) based on the determined at least one split location (182).

2. The method according to claim 1,
wherein the at least one fibrotic segment (160) is traversed by one or more conductive channels (172); and
wherein the at least one split location (182) is indicative of a location for the simulated wave entering into or exiting from one of the one or more conductive channels (172) in the fibrotic segment (170).

3. The method according to any one of the preceding claims, wherein determining the one or more target sites (200) based on the determined at least one split location (182) further comprises simulating evolution of at least one further cardiac activation wave unidirectionally excited at the at least one split location (182).

4. The method according to claim 3,
wherein a plurality of split locations (182) is determined; and
wherein for each of the split locations (182) at least one further cardiac activation wave unidirectionally excited at the respective split location is simulated.

5. The method according to any one of the preceding claims,
wherein a plurality of split locations (182) is determined; and
wherein the method further comprises discarding at least one of the determined split locations (182) based on determining that said at least one split location (182) is arranged between two neighboring split locations on opposing sides of said at least one split location (182).

6. The method according to any one of claims 3 to 5, further comprising:
determining a round trip time based on the simulated at least one further cardiac activation wave, the round trip time being indicative of a travel time of the at least one further cardiac activation wave along an electric circuit (195) between initiation on one side of the at least one split location (182) and arrival at an opposite side of the at least one split location (182); and/or
determining a round trip distance based on the simulated at least one further cardiac activation wave, the round trip distance being indicative of a length of an electric circuit (195), along which the at least one further activation wave travels from one side of the at least one split location (182) to an opposite side of the at least one split location (182).

7. The method according to claim 6, further comprising:
comparing the determined round trip time and/or the determined round trip distance to a threshold value; and
selecting the at least one split location (182) as target site (200) if the determined round trip time and/or the determined round trip distance reaches or exceeds the threshold value.

8. The method according to any one of claims 6 and 7,
wherein a plurality of split locations (182) is determined and for each of the determined split locations a round trip time and/or a round trip distance is determined; and
wherein the at least one target site is determined based on selecting the split location (182) according to one or more criteria related to the round trip time and/or the round trip distance.

9. The method according to any one of the preceding claims,
wherein the cardiac activation wave and/or at least one further cardiac activation wave is simulated based on stimulating spatio-temporal evolution of the respective activation wave at one or more stimulus sites (190) in the cardiac model (150); and/or
wherein the cardiac activation wave and/or at least one further cardiac activation wave is simulated based on a wavefront propagation model.

10. The method according to any one of 3 to 9,
wherein a plurality of split locations (182) is determined and wherein the method further comprises:
determining, for each of the determined split locations (182), an activation time map indicative of a spatio-temporal evolution of the simulated at least one further wave across the cardiac model (150).

11. The method according to claim 10, further comprising:
computing a correlation coefficient between temporally aligned activation time maps associated with different split locations (182); and
determining the one or more target sites (200) based on comparing the computed correlation coefficients to a threshold value.

12. The method according to any one of the preceding claims, further comprising:
generating an updated cardiac model based on permanently blocking traversal of a cardiac activation wave at the target site; and
repeating at least steps S1 and S2 using the updated cardiac model.

13. A computer program, which, when executed on a processor (110) of a computing system (100), instructs the computing system (100) to carry out steps of the method according to any one of the preceding claims.

14. A non-transitory computer-readable medium storing a computer program according to claim 13.

15. A computing system (100) with one or more processors (110), wherein the computing system (100) is configured to carry out steps of the method according to any one of claims 1 to 13.

## Patentansprüche

1. Computerimplementiertes Verfahren zur Bestimmung einer oder mehrerer Zielstellen (200) zur Planung der Behandlung von ventrikulärer Tachykardie, wobei das Verfahren umfasst:
Empfangen, mit einer Rechenvorrichtung (100), die einen oder mehrere Prozessoren (110) umfasst, dreidimensionale Modelldaten, die ein Herzmodell (150) beschreiben, welches die Anatomie eines Herzens eines Patienten mit Fibrose modelliert, wobei das Herzmodell (150) mindestens ein Myokardsegment (160), das mit elektrisch leitendem Myokardgewebe assoziiert und/oder als solches modelliert ist, und mindestens ein fibrotisches Segment (170), das mit isolierendem fibrotischem Gewebe assoziiert und/oder als solches modelliert ist, umfasst;
Simulieren (S1), durch die Rechenvorrichtung (100) unter Verwendung des Herzmodells (150), der Entwicklung einer Herzaktivierungswelle über das mindestens eine Myokardsegment (160) und das mindestens eine fibrotische Segment (170);
**dadurch gekennzeichnet, dass** das Verfahren ferner umfasst:
Bestimmen (S2) mindestens einer Teilungsstelle (182), an der eine Isoschicht (180) der simulierten Welle in zwei oder mehr Abschnitte (18a-c) geteilt wird, wobei die mindestens eine Teilungsstelle (182) eine Stelle angibt, an der die simulierte Welle auf eine Grenze zwischen dem mindestens einen fibrotischen Segment (170) und dem mindestens einen Myokardsegment (160) trifft; und
Bestimmen einer oder mehrerer Zielstellen (200) auf der Grundlage der bestimmten mindestens einen Teilungsstelle (182).

2. Verfahren nach Anspruch 1,
wobei das mindestens eine fibrotische Segment (170) von einem oder mehreren leitfähigen Kanälen (172) durchzogen ist; und
wobei die mindestens eine Teilungsstelle (182) eine Stelle angibt, an der die simulierte Welle in einen der einen oder mehreren leitfähigen Kanäle (172) im fibrotischen Segment (170) eintritt oder aus diesem austritt.

3. Verfahren nach einem der vorstehenden Ansprüche, wobei das Bestimmen der einen oder mehreren Zielstellen (200) auf der Grundlage der ermittelten mindestens einen Teilungsstelle (182) ferner das Simulieren der Entwicklung mindestens einer weiteren Herzaktivierungswelle umfasst, die an der mindestens einen Teilungsstelle (182) unidirektional angeregt wird.

4. Verfahren nach Anspruch 3,
wobei eine Vielzahl von Teilungsstellen (182) bestimmt wird; und
wobei für jede der Teilungsstellen (182) mindestens eine weitere Herzaktivierungswelle simuliert wird, die an der jeweiligen Teilungsstelle unidirektional angeregt wird.

5. Verfahren nach einem der vorstehenden Ansprüche,
wobei eine Vielzahl von Teilungsstellen (182) bestimmt wird; und
wobei das Verfahren ferner das Verwerfen mindestens einer der ermittelten Teilungsstellen (182) umfasst, basierend auf der Feststellung, dass die mindestens eine Teilungsstelle (182) zwischen zwei benachbarten Teilungsstellen auf gegenüberliegenden Seiten der mindestens einen Teilungsstelle (182) angeordnet ist.

6. Verfahren nach einem der Ansprüche 3 bis 5, das ferner umfasst:
das Bestimmen einer Umlaufzeit auf der Grundlage der simulierten mindestens einen weiteren Herzaktivierungswelle, wobei die Umlaufzeit eine Laufzeit der mindestens einen weiteren Herzaktivierungswelle entlang eines elektrischen Stromkreises (195) zwischen dem Start auf einer Seite der mindestens einen Teilungsstelle (182) und der Ankunft an einer gegenüberliegenden Seite der mindestens einen Teilungsstelle (182) angibt; und/oder
Bestimmen einer Umlaufstrecke auf der Grundlage der simulierten mindestens einen weiteren Herzaktivierungswelle, wobei die Umlaufstrecke eine Länge eines elektrischen Stromkreises (195) angibt, entlang dem die mindestens eine weitere Aktivierungswelle von einer Seite der mindestens einen Teilungsstelle (182) zu einer gegenüberliegenden Seite der mindestens einen Teilungsstelle (182) verläuft.

7. Verfahren nach Anspruch 6, das ferner umfasst:
Vergleichen der ermittelten Umlaufzeit und/oder der ermittelten Umlaufstrecke mit einem Schwellenwert; und
Auswählen der mindestens einen Teilungsstelle (182) als Zielstelle (200), wenn die ermittelte Umlaufzeit und/oder die ermittelte Umlaufstrecke den Schwellenwert erreicht oder überschreitet.

8. Verfahren nach einem der Ansprüche 6 und 7,
wobei eine Vielzahl von Teilungsstellen (182) bestimmt wird und für jede der bestimmten Teilungsstellen eine Umlaufzeit und/oder eine Umlaufstrecke bestimmt wird; und
wobei die mindestens eine Zielstelle auf der Grundlage der Auswahl der Teilungsstelle (182) gemäß einem oder mehreren Kriterien bestimmt wird, die sich auf die Umlaufzeit und/oder die Umlaufstrecke beziehen.

9. Verfahren gemäß einem der vorstehenden Ansprüche,
wobei die Herzaktivierungswelle und/oder mindestens eine weitere Herzaktivierungswelle auf der Grundlage des Stimulierens der räumlich-zeitlichen Entwicklung der jeweiligen Aktivierungswelle an einer oder mehreren Stimulationsstellen (190) im Herzmodell (150) simuliert wird; und/oder
wobei die Herzaktivierungswelle und/oder mindestens eine weitere Herzaktivierungswelle auf der Grundlage eines Wellenfrontausbreitungsmodells simuliert wird.

10. Verfahren gemäß einem der Ansprüche 3 bis 9,
wobei eine Vielzahl von Teilungsstellen (182) bestimmt wird und wobei das Verfahren ferner umfasst:
das Bestimmen einer Aktivierungszeitkarte für jede der ermittelten Teilungsstellen (182), die eine räumlich-zeitliche Entwicklung der simulierten mindestens einen weiteren Welle über das Herzmodell (150) hinweg angibt.

11. Verfahren nach Anspruch 10, das ferner umfasst:
Berechnen eines Korrelationskoeffizienten zwischen zeitlich abgeglichenen Aktivierungszeitkarten, die verschiedenen Teilungsstellen (182) zugeordnet sind; und
Bestimmen der einen oder mehreren Zielstellen (200) auf der Grundlage eines Vergleichs der berechneten Korrelationskoeffizienten mit einem Schwellenwert.

12. Verfahren nach einem der vorstehenden Ansprüche, das ferner umfasst:
Erzeugen eines aktualisierten Herzmodells auf der Grundlage einer dauerhaft blockierten Ausbreitung einer Herzaktivierungswelle an der Zielstelle; und
Wiederholen zumindest der Schritte S1 und S2 unter Verwendung des aktualisierten Herzmodells.

13. Computerprogramm, das, wenn es auf einem Prozessor (110) eines Computersystems (100) ausgeführt wird, das Computersystem (100) anweist, Schritte des Verfahrens gemäß einem der vorstehenden Ansprüche auszuführen.

14. Nicht-flüchtiges, computerlesbares Medium, das ein Computerprogramm gemäß Anspruch 13 speichert.

15. Computersystem (100) mit einem oder mehreren Prozessoren (110), wobei das Computersystem (100) so konfiguriert ist, dass es Schritte des Verfahrens gemäß einem der Ansprüche 1 bis 13 ausführt.

## Revendications

1. Procédé mis en œuvre par ordinateur pour déterminer un ou plusieurs sites cibles (200) pour la planification du traitement d'une tachycardie ventriculaire, le procédé comprenant :
la réception, à l'aide d'un dispositif informatique (100) comprenant un ou plusieurs processeurs (110), des données de modèle tridimensionnel représentatives d'un modèle cardiaque (150) modélisant l'anatomie du coeur d'un sujet présentant une fibrose, dans lequel le modèle cardiaque (150) comprend au moins un segment myocardique (160) associé à et/ou modélisé comme du tissu myocardique électriquement conducteur et au moins un segment fibrotique (170) associé à et/ou modélisé comme du tissu fibrotique isolant ;
la simulation (S1), par le dispositif de calcul (100) à l'aide du modèle cardiaque (150), de l'évolution d'une onde d'activation cardiaque à travers le au moins un segment myocardique (160) et le au moins un segment fibrotique (170) ;
**caractérisé en ce que** le procédé comprend en outre :
la détermination (S2) d'au moins un emplacement de division (182), au niveau duquel une isosurface (180) de l'onde simulée est divisée en deux sections ou plus (18a-c), ledit au moins un emplacement de division (182) indiquant un emplacement où l'onde simulée frappe une limite entre ledit au moins un segment fibrotique (170) et ledit au moins un segment myocardique (160) ; et
la détermination d'un ou plusieurs sites cibles (200) sur la base de l'au moins un emplacement de division déterminé (182).

2. Procédé selon la revendication 1,
dans lequel le ou les segments fibrotiques (170) sont traversés par un ou plusieurs canaux conducteurs (172) ; et
dans lequel l'au moins un emplacement de division (182) indique un emplacement où l'onde simulée entre dans ou sort de l'un des un ou plusieurs canaux conducteurs (172) dans le segment fibrotique (170).

3. Procédé selon l'une quelconque des revendications précédentes, dans lequel la détermination du ou des sites cibles (200) sur la base de l'au moins un emplacement de division (182) déterminé comprend en outre la simulation de l'évolution d'au moins une autre onde d'activation cardiaque excitée de manière unidirectionnelle au niveau de l'au moins un emplacement de division (182).

4. Procédé selon la revendication 3,
dans lequel une pluralité d'emplacements de division (182) est déterminée ; et
dans lequel, pour chacun des emplacements de division (182), au moins une autre onde d'activation cardiaque excitée de manière unidirectionnelle au niveau de l'emplacement de division respectif est simulée.

5. Procédé selon l'une quelconque des revendications précédentes,
dans lequel une pluralité d'emplacements de division (182) est déterminée ; et
dans lequel le procédé comprend en outre l'élimination d'au moins l'un des emplacements de division déterminés (182) sur la base de la détermination que ledit au moins un emplacement de division (182) est situé entre deux emplacements de division voisins sur les côtés opposés dudit au moins un emplacement de division (182).

6. Procédé selon l'une quelconque des revendications 3 à 5, comprenant en outre :
la détermination d'un temps aller-retour sur la base de la ou des ondes d'activation cardiaque supplémentaires simulées, le temps aller-retour étant indicatif d'un temps de propagation de la ou des ondes d'activation cardiaque supplémentaires le long d'un circuit électrique (195) entre le déclenchement d'un côté de l'emplacement de division (182) et l'arrivée à un côté opposé dudit emplacement de division (182) ; et/ou
la détermination d'une distance aller-retour sur la base de la ou des ondes d'activation cardiaque supplémentaires simulées, la distance aller-retour étant indicative de la longueur d'un circuit électrique (195) le long duquel la ou les ondes d'activation supplémentaires se propagent d'un côté de l'emplacement de division (182) à un côté opposé de l'emplacement de division (182).

7. Procédé selon la revendication 6, comprenant en outre :
la comparaison du temps aller-retour déterminé et/ou de la distance aller-retour déterminée à une valeur seuil ; et
la sélection de l'au moins un emplacement de division (182) comme site cible (200) si le temps aller-retour déterminé et/ou la distance aller-retour déterminée atteint ou dépasse la valeur seuil.

8. Procédé selon l'une quelconque des revendications 6 et 7,
dans lequel on détermine une pluralité d'emplacements de division (182) et, pour chacun des emplacements de division déterminés, on détermine un temps aller-retour et/ou une distance aller-retour ; et
dans lequel le au moins un site cible est déterminé sur la base de la sélection de l'emplacement de division (182) selon un ou plusieurs critères liés au temps aller-retour et/ou à la distance aller-retour.

9. Procédé selon l'une quelconque des revendications précédentes,
dans lequel l'onde d'activation cardiaque et/ou au moins une autre onde d'activation cardiaque est simulée sur la base de la stimulation de l'évolution spatio-temporelle de l'onde d'activation respective au niveau d'un ou plusieurs sites de stimulation (190) dans le modèle cardiaque (150) ; et/ou
dans lequel l'onde d'activation cardiaque et/ou au moins une autre onde d'activation cardiaque est simulée sur la base d'un modèle de propagation du front d'onde.

10. Procédé selon l'une quelconque des revendications 3 à 9,
dans lequel une pluralité d'emplacements de division (182) est déterminée et dans lequel le procédé comprend en outre :
la détermination, pour chacun des emplacements de division déterminés (182), d'une carte de temps d'activation indicative d'une évolution spatio-temporelle de la au moins une autre onde simulée à travers le modèle cardiaque (150).

11. Procédé selon la revendication 10, comprenant en outre :
le calcul d'un coefficient de corrélation entre des cartes de temps d'activation alignées dans le temps associées à différents emplacements de division (182) ; et
la détermination du ou des sites cibles (200) sur la base d'une comparaison des coefficients de corrélation calculés avec une valeur seuil.

12. Procédé selon l'une quelconque des revendications précédentes, comprenant en outre :
la génération d'un modèle cardiaque mis à jour sur la base du blocage permanent de la propagation d'une onde d'activation cardiaque au niveau du site cible ; et
la répétition d'au moins les étapes S1 et S2 en utilisant le modèle cardiaque mis à jour.

13. Programme informatique qui, lorsqu'il est exécuté sur un processeur (110) d'un système informatique (100), ordonne au système informatique (100) d'exécuter les étapes du procédé selon l'une quelconque des revendications précédentes.

14. Support non transitoire lisible par ordinateur stockant un programme informatique selon la revendication 13.

15. Système informatique (100) comportant un ou plusieurs processeurs (110), dans lequel le système informatique (100) est configuré pour exécuter les étapes du procédé selon l'une quelconque des revendications 1 à 13.
